Europäisches Patentamt

**European Patent Office**

**Office européen des brevets**

(19)

(11) Publication number: **0 002 452**
**B1**

(12) **EUROPEAN PATENT SPECIFICATION**

(45) Date of publication of patent specification: **30.03.83**

(51) Int. Cl.³: **C 07 G 7/00** //A61K37/02

(21) Application number: **78101416.2**

(22) Date of filing: **20.11.78**

(54) Glycoprotein isolated from aloe.

(30) Priority: **21.11.77 JP 138907/77**
**25.02.78 JP 20402/78**

(43) Date of publication of application:
**27.06.79 Bulletin 79/13**

(45) Publication of the grant of the patent:
**30.03.83 Bulletin 83/13**

(84) Designated Contracting States:
**CH DE FR GB NL SE**

(56) References cited:
**DERWENT JAPANESE PATENTS REPORT, vol.
S, no. 33, (1971)**

(73) Proprietor: **Aloace Co., Ltd.**
**No. 20-19 Marunouchi 2-chome Naka-ku
Nagoya-shi (JP)**

(72) Inventor: **Suzuki, Ikuo**
**No. 2874-115, Minamiyama Nagasaka-cho
Owariasahi-shi Aichi-ken (JP)**

(74) Representative: **Strehl, Peter et al,
Strehl, Schübel-Hopf, Schulz Patentanwälte
Widenmayerstrasse 17
D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 002 452

## Glycoprotein isolated from aloe

This invention relates to a novel substance Aloctin A isolated from Aloe, a plant belonging to *Liliaceae*. The novel substance demonstrates several biological activities and therefore is expected to be used as medicine for diseases such as cancer, inflammation, burn and skin diseases.

It was known that a substance named Alomicin having anti-tumor activity may be obtained from lyophilized extract of Cape Aloe by fractionating the extract with ethanol in two steps and following $Pb(OAc)_2$ and Sephadex G-25-gel filtration. The use of Sephadex G-25-gel filtration indicates that the substance should have a low molecular weight of less than 5000. (Chemical Abstracts, Vol. 73, 1970, 75501e).

The novel substance Aloctin A is however a rather high molecular substance, having the following characteristics:

(1) Molecular weight of the material is approximately $1.8 \times 10^4$.

(2) The material is a glycoprotein in which ratio of protein to suger is 8 to 2 by weight.

(3) In sodium dodecyl sulphate-polyacrylamide (abbreviated as SDS-polyacrylamide) gel electrophoresis, the material gives a single band without 2-mercaptoethanol treatment and gives two discrete bands with 2-mercaptoethanol treatment, by which S-S bonding of the material is cleaved.

(4) The material has hemagglutinating and cytoagglutinating activity for transformed cells.

(5) The material has mitogenic activity for lymphocytes.

(6) The material reacts with serum proteins of mammalians, fish, amphibia and reptilia and forms precipitin lines in agarose gel plates.

(7) The material has activating activity of complement third component (C3) which is $\beta$-globulin in serum and is one of the main factors of the immuno reaction.

Aloctin A in the present invention can be isolated from Aloe, a plant belonging to *Liliaceae* such as *Aloe arborescens* MILL, *Aloe perryi* BAKER, Aloe barbadensis MILLER and *Aloe forox* MILLER. The material is present in all parts of the plant, i.e., leaves, trunks, bark, seeds and others, and can be isolated therefrom.

Isolation and purification

An example of isolating method is as follows: The juice of 1000 g crushed *Aloe arborescens* MILL leaves was filtered through gauze and then centrifuged at 10 000 rpm for 30 min. Coarse materials were removed thereby. The clear supernatant was subjected to ammonium sulfate fractionation. The fraction precipitated by 0—40% saturation with ammonium sulfate was dissolved in 0.05 M carbonate-bicarbonate buffer (pH 9.5) and dialyzed overnight against the same buffer to remove ammonium sulfate. The solution was lyophilized.

The dialyzed material thus obtained, which is referred to AS-40 hereinafter, demonstrated hemagglutinating activity. A small amount of 1 M acetic acid was added to the AS-40 solution in 0.05 M carbonate-bicarbonate buffer (pH 9.5) to give a pH of 4.4. Because of change in pH, the formerly clear AS-40 solution became cloudy. The cloudy solution was centrifuged at 10 000 rpm for 20 minutes. The supernatant, which is referred to as Acidic sup hereinafter, was isolated from the precipitate, which is referred to as Acidic ppt hereinafter. Higher hemagglutinating activity was detected in Acidic sup than in Acidic ppt. Mitogenic activity for lymphocytes was detected only in Acidic ppt.

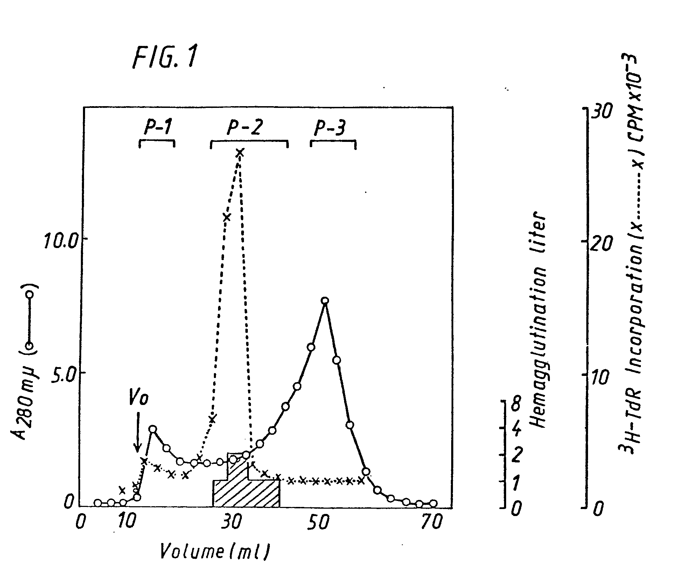

The Acidic ppt was dissolved in 0.05 M phosphate buffer (pH 8.0) after lyophylization and filtered on a Sephadex G-200 (produced by Pharmacia Fine Chemical, Sweden) Column (1.5×25 cm), previously equilibrated with the same buffer. Elution was carried out at 4° C with the same buffer at the flow rate of 2 ml per hour. Fractions of 1.3 ml of eluent were collected. Elution curve of protein by $A_{280m\mu}$ is shown in Fig. 1. The elution volumes of Blue Dextran was determined and is indicated by vertical arrow (Vo). Hemagglutinating activity is denoted by shaded portions and mitogenic activity is denoted by dotted line.

The hemagglutinating and mitogenic activities were detected in fractions from 27.3 ml to 44.2 ml eluent (P-2). These biological activities were not detected in other fractions (P-1 and P-3). The fraction from 27.3 ml to 44.2 ml eluent (P-2) contains the Aloctin A of the present invention.

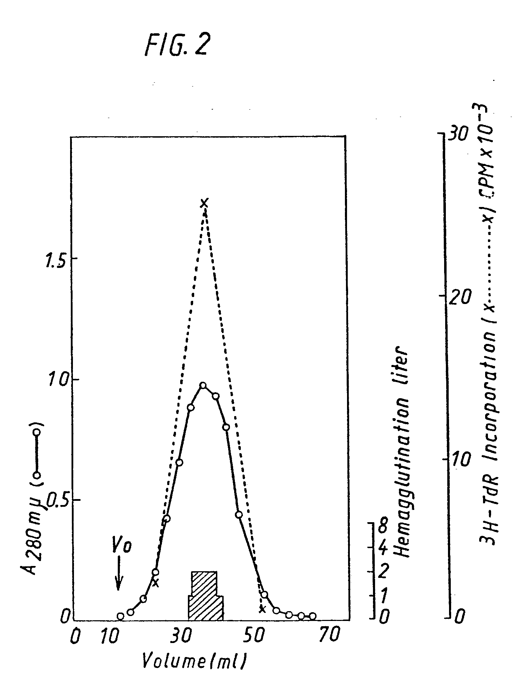

This fraction was further purified by rechromatography. The fraction from 27.3 to 44.2 ml eluent (1.2 mg) was collected together in Spectrapor membrane tube (Spectrum Medical Industries, Inc. Calf., U.S.A.), and condensed by covering the membrane tube with polyethylenglycol. The condensed solution was re-filtered on the column (1,5×25 cm) of Sephadex G-200, previously equilibrated with 0.05 M phosphate buffer (pH 8.0). Elution was carried out at 9°C with 0.05 M phosphate buffer (pH 8.0) at a flow rate of 2 ml/h, and fractions of 1.3 ml each were collected. The result was shown in Fig. 2. (vo: void volume). Thus purified material is Aloctin A of the present invention.

An example of yield of protein, hemagglutinating and mitogenic activity in each fraction during the purification process is summarized in Table I.

2

TABLE I

Summary of purification of Aloctin A from 1000 g of Aloe Leaves

| Fraction | Total protein | HA* activity[a] | HA* titer | HA* activity recovered | Mitogenic activity[b] |
|---|---|---|---|---|---|
| | mg | µg/ml | (per mg protein) | % | µg/ml |
| Juice | n.d.* | 500 | 2 | | n.d.** |
| AS-40 | 320.0 | 250 | 4 | 100 | n.d.** |
| Acidic ppt | 47.4 | 250 | 4 | 14.8 | 30.0 |
| P-1 | 1.05 | >1.000 | 0 | 0 | >100 |
| P-2 | 2.6 | 62.5 | 16 | 3.3 | 5.0 |
| Acidic sup | 18.4 | 125 | 8 | 11.5 | >100 |

[a]Minimum hemagglutinating dose against human erythrocytes

[b]Mitogenic dose to give 20 000 cpm of [methyl- $^3$H]thymidine incorporation against $1 \times 10^6$ lymphocytes

*Hemagglutination

**Not determined

Chemical and physical properties
Chemical Analysis
   Aloctin A was positive in following qualitative protein and carbohydrate reactions: Ninhydrin reaction, Xantho-protein reaction, Molisch-Udranszky reaction, Anthrone reaction, Fehling reaction, Elson-Morgan reaction and Periodic acid-Schiff reaction. Quantitative analysis showed that Aloctin A contained 18.3% neutral carbohydrate, suggesting that Aloctin A is a glycoprotein.

Elemental Analysis
       C: 42.0—51—4 (%), H: 5.7—7.0 (%),
       N: 13.4—16.4 (%), O: 20.2—24.6 (%).

IR-Spectrum

TABLE II

| Wave number | Strength of absorbance |
|---|---|
| 1200—1240 cm$^{-1}$ | medium |
| 1500—1520 | strong |
| 1620—1640 | strong |
| 3200—3260 | strong |

$^{13}$C-NMR spectrum

TABLE III

| Chemical shift | Spectral pattern |
|---|---|
| 15—27 ppm | broad, multiplet |
| 67—69 | sharp, singlet |
| 71—77 | sharp, multiplet |
| 99—100 | sharp, singlet |
| 169—180 | broad, multiplet |

Optical rotation
   Optical rotation measurement was conducted at 470 nm with a cell 1 cm in length. A 7.7 mg/ml (calculated as bovine serum albumin) solution of Aloctin A in 0.05 M phosphate buffer (pH 8.0) showed a negative optical rotation at 25° C.

Disc electrophoresis
   Sodium dodecyl sulfate (SDS) polyacrylamide gel electrophoresis was carried out according to the method of Weber and Osborn (Weber, K., and Osborn, M. (1969) J. Biol. Chem. 244, 4406—4412). Experiments were performed with samples some of which had been pre-treated with 2-mercaptoethanol. For estimation of molecular weight, the method of Segrest and Jackson (Segrest, J. P., Jackson, R. L. (1972 in Methods in Enzymology, (Ginsburg, V. ed.) Vol. 28 (B), p. 54—63, Acad. Press, New York) was followed, using various concentrations of gel from 5% to 20.0%. As molecular weight standards, bovine serum albumin (BSA), ovalbumin, chymotrypsinogen A and cytochrome C were used. The gels were normally stained for protein with Coomassie brilliant blue and destained with a mixture of 10% acetic acid and 10% isopropanol.

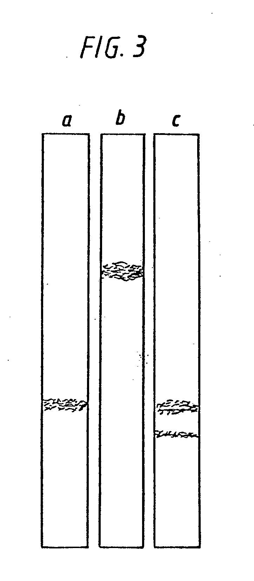

SDS-polyacrylamide gel electrophoresis in the 10% gel of the material isolated from P-1 fractions and Aloctin A without 2-mercaptoethanol treatment gave a single band as shown in Fig. 3 a and b. The material isolated from P-1 fractions gave a same single band either with or without 2-mercaptoethanol treatment. Aloctin A, after 2-mercaptoethanol treatment, gave two discrete bands, a smaller peptide band ($\alpha$) and a larger peptide band ($\beta$) (Fig. 3 c).

From these results it has been concluded that $\alpha$ and $\beta$ peptides were subunits of Aloctin A.

Stated in detail the SDS-polyacrylamide gel electrophoresis was carried out with 10% gel in 0.1 M sodium phosphate buffer, pH 7.2, containing 0.1% SDS. Samples were heated at 100° C for 5 min in 0.01 M sodium phosphate buffer, pH 7.2, containing 1% SDS, 25% glycerol, and 0.001% bromphenol blue with and without 5% 2-mercaptoethanol.

a: Aloctin A with and without 2-mercaptoethanol treatment (the same result was obtained), b: Aloctin A without 2-mercaptoethanol treatment, c: Aloctin A with 2-mercaptoethanol treatment d: S-1.

Molecular weight estimation

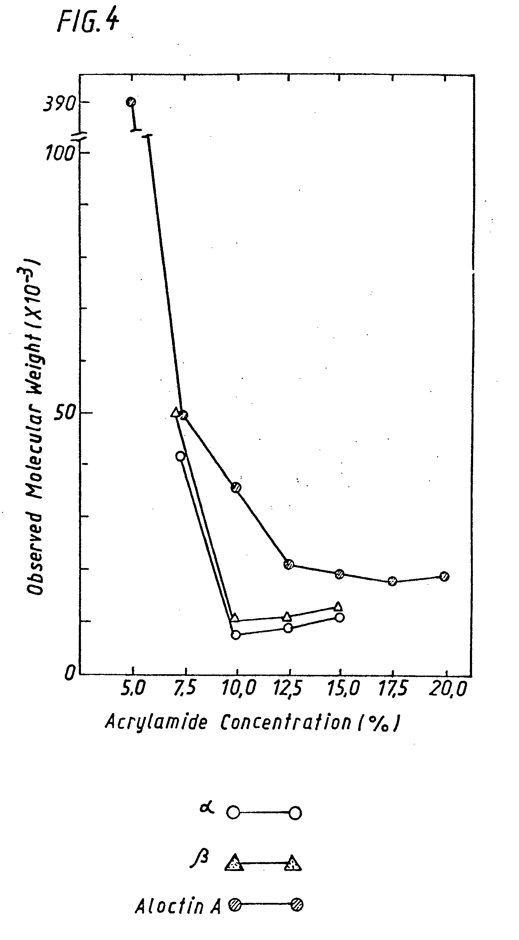

To obtain the molecular weight of Aloctin A, the method by Segret and Jackson was adopted using various concentrations of gel such as 5.0, 7.5, 10.0, 12.5, 15.0, 17.5 and 20.0%. SDS-polyacrylamide gel electrophoresis was carried out as described in Fig. 3. The mobility of protein was measured and the relationship between the logarithm of the molecular weights of several marker proteins and their mobilities was plotted. Molecular weight markers (molecular weights shown in parenthesis) used were: cytochrome c (12 500), chymotrypsinogen A (25 000), ovalbumin (45 000), bovine serum albumin (67 000). The result was shown in Fig. 4. An asymptotic minimal molecular weight could be estimated from each curve in Fig. 4. Following values have been obtained: 18 000 for Aloctin A, 7 500 for $\alpha$, and 10 500 for $\beta$, although these values were not complete because of lack of correction for the total amount of carbohydrate in the glycoprotein molecule.

Amino acid and sugar analysis

Samples were hydrolyzed in 6N HCl in evacuated, sealed tubes at 110°C for 20 h. The amino acid content of the hydrolysates was determined in a Hitachi amino acid analyzer (KLA-3B) according to the method of Spackman et al. (Spackman, D. H., Stein, W. H., and Moore, S. (1958) Anal, Chem. *30*, 1190—1206). Cysteine and cystine were identified as S-sulfocysteine by the method of Liu et al. (Liu, T. Y. and Inglis A. S. (1972 in Methods of Enzymology (Hirs, C. H. W., and Timasheff, S. N. eds.) Acad. Press, New York Vol 25, p. 55—60). Tryptophan was not determined. The total amount of neutral sugars was determined by the orcinol-$H_2SO_4$ procedure. (Winzler, R. J. (1956) in Method of Biochemical Analysis (Glick, D., ed.) Vol. 2, p. 279-311 Interscience, New York).

The amino acid composition of Aloctin A is shown in Table IV.

In this experiment tryptophan was not determined. The most notable feature of the amino acid composition is a high proportion of acidic amino acids such as aspartic acid and glutamic acid and a low proportion of methionine and histidine.

Quantitative analysis showed that Aloctin A contained 18.3% of neutral carbohydrate, suggesting that Aloctin A is glycoprotein.

TABLE IV
Amino acid composition of Aloctin A and β-subunit

| Amino acid | Aloctin A | β-subunit |
|---|---|---|
| | mole/mole of protein[a] | |
| Aspartic acid | 27.50 (14)[b] | 7.93 (8)[b] |
| Threonine | 17.64 (9) | 3.63 (4) |
| Serine | 19.76 (10) | 4.57 (5) |
| Glutamic acid | 33.36 (17) | 7.51 (8) |
| Proline | 15.60 (8) | 4.47 (4) |
| Glycine | 30.68 (15) | 6.88 (7) |
| Alanine | 19.96 (10) | 6.72 (7) |
| Half-cystine | 7.94 (4) | 2.28 (2) |
| Valine | 18.16 (9) | 5.31 (5) |
| Methionine | 2.48 (1) | 1.18 (1) |
| Isoleucine | 14.64 (7) | 4.11 (4) |
| Leucine | 25.20 (13) | 6.61 (7) |
| Tyrosine | 9.80 (5) | 2.35 (2) |
| Phenylalanine | 6.12 (3) | 2.90 (3) |
| Lysine | 7.12 (4) | 3.79 (4) |
| Histidine | 5.68 (3) | 1.40 (1) |
| Arginine | 9.04 (5) | 2.64 (3) |

[a]Molecular weight of Aloctin A was taken as 18 000.
[b]Number of residues to the nearest integer.

Biological properties
Hemo- and cytoagglutinating activity

Hemagglutination Titration: Hemagglutination titration was carried out with microtiter equipment (Cooke Lab. Prod., Virginia, U.S.A.). Human erythrocytes were treated with 0.1% trypsin for 1 H, washed three times with phosphate buffered saline and a 2% suspension was used for assay. For the assay, 25 μl aliquots of 2-fold serial dilution of lectin solution were mixed with an equal volume of erythrocyte suspension and left for 1 h at room temperature with occasional shaking. The degree of agglutination was evaluated macroscopically and the titer value was  defined as titer/mg protein.

Hemagglutinating Activity: Aloctin A agglutinates erythrocytes of various species such as human, sheep and rabbit, and does not show A-B-O blood group specificity in hemagglutination tests in the human system. Treatment of human erythrocytes with 0.1% trypsin increases the agglutination approximately 5-fold compared with non-treated erythrocytes. The minimum hemagglutinating doses of the purified fractions on trypsin-treated human erythrocytes are shown in Table I.

Cytoagglutinating Activity: Cytoagglutination titration was carried out in the same manner to hemagglutination titration in comparison with concanavalin A (Con A). The result was shown in Table V. As shown in this Table, Aloctin A showed the analogous cytoagglutination titer as Con A against various cell lines. However, quite high titer was recorded with Aloctin A in X-ray induced thymoma. Generally highly malignant transformed cell lines, such as P3-J, Molt-4B, X-ray induced thymoma, KB, HeLa S-3 and CHO-KI were higher in cytoagglutination titer than those with lower malignancy or normal nature (not malignant), such as NC-37, DON-6, NR-K and NS-2.

# 0 002 452

### TABLE V
### Cytoagglutination titer

| Cells | Con A | | Aloctin A | |
|---|---|---|---|---|
| NC-37 | 4 | | 4 | |
| P3-J | 32 | | 32 | |
| P3HR-1 | 32 | | 32 | |
| Molt-4B | 32 | | 32 | |
| M. Spleen Cell | 32 | | 32 | |
| Xray-thymoma | 64 | | 256 | |
| | T-E* | E** | T-E* | E* |
| KB | 32 | 16 | 32 | 16 |
| HeLa-S3 | 32 | 16 | 32 | 16 |
| Don-6 | 4 | 2 | 1 | 1 |
| CHO-$K_1$ | 16 | 16 | 64 | 32 |
| HNG-100 | 32 | 32 | 64 | 32 |
| NR-K | 16 | 4 | 8 | 4 |
| NS-2 | 4 | 2 | 8 | 4 |
| REWK$A_2$ | 16 | 16 | 16 | 16 |

*Treatment with the mixture of 0,05% trypsin and 0.02% EDTA
**Treatment with 0.02% EDTA

Mitogenic Activity

Preparation of Human Lymphocytes: Normal human venous blood was withdrawn into syringes previously treated with heparin. Purified lymphocytes were obtained by the method of Kawaguchi et al. (Kawaguchi, T., Matsumoto, I., and Osawa, T. (1973), J. Biol. Chem. *249*, 2786—2792). The cells were then washed with 0.25% bovine serum albumin in 0.15 M NaCl—0.01 M phosphate buffer (pH 7) and used for examination of mitogenic activity.

Assay of Mitogenic Activity: Morphological examination of lymphocyte transformation was carried out by determining the percentage of transformed cells in a Giemsa-stained preparation, counting approximately 1 000 cells per mitogen sample.

Assay of [$^3$H]thymidine incorporation was performed by adding [methyl-$^3$H]thymidine (2.0 $\mu$Ci, The Radiochemical Center, England) to each tube ($1 \times 10^6$ lymphocytes/ml, 2 ml) after the incubation with mitogen sample for six hours. Sixty hours after the addition of [methyl-$^3$H]thymidine, the cells were collected and washed three times with cold phosphate buffered saline (pH 7.2). After adding 1.5 ml of cold 5% trichloro acetic acid and 2 ml cold methanol to the cell pellet, one drop of 3% bovine serum albumin (BSA) as carrier protein was added. The precipitate was washed on a membrane (GF/C 2.5 cm, Whatman) in a "Manifold" multiple sample collector (Millipore). To dried membranes, 0.3 ml solubilizer (Soluene TM 100, Packard) was added and incubated at 37°C for 2 hours, and 5 ml of scintillation fluid (5 g 2,5-diphenyloxazole and 0.1 g 1,4-bis-2-(5-phenyloxazolyl)benzene in 1 000 ml toluene were added. The radioactivity was counted in a Beckman LS-200B liquid scintillation counter.

Seventy-two hours after the culture of human lymphocytes with Aloctin A or Acidic ppt, large lymphocytes with morphologically transformed shapes were abundant compared to culture systems with purified $\beta$-subunit and without mitogen. When approximately 1 000 cells were counted, about 70% of the lymphocytes were found to be transformed after 72 hours culture. This degree of transformation is almost the same as that by phytohemagglutinin-W (PHA-W, Wellcome Co., U.S.A.) which was tested as a positive control.

In order to demonstrate [methyl-$^3$H]thymidine incorporation as a function of the mitogen, lymphocytes were treated with various quantities of Aloe fractions such as Acidic ppt, $\beta$-subunit and Aloctin A.

As shown in Table I, mitogenic activity was detected in both Acidic ppt and Aloctin A. Among the purified fractions Aloctin A alone displayed mitogenic activity.

Cap forming activity on various cell membranes

Corelated with mitogenic activity, cap forming activity of Aloctin A on membranes of lymphocytes and other various cell lines were examined in comparison with Con A.

Fluorescence-labelled Aloctin A (FITC-Aloctin A) was prepared by the method of Cebra and Goldstein (Cebra, J. J. & Goldstein, G. (1965) J. Immunol., *95*, 230—245). Cells were stained with FITC-Aloctin A at 37° C for 40 min, after washing cells with phosphate buffered saline and were observed by fluorescence microscopy. The result was shown in Table VI. As shown in this table it is revealed that Aloctin A has cap forming activity on not only lymphocytes but also various cultured cells.

6

TABLE VI

| Cells | Ratio of cells with caps (%) | |
|---|---|---|
| | FITC-Con A | FITC-Aloctin A |
| N.H.P.L* | | 35 |
| P3HR-1 | 14 | 25 |
| Molt-4B | 20 | 49 |
| KB | 12 | 60 |
| HeLa-S3 | 30 | 45 |
| Don-6 | 0 | 0 |
| CHO-K$_1$ | 0 | 30 |
| HNG-100 | 0 | 37 |
| NR-K | 0 | 6 |
| NS-2 | 12 | 31 |
| REWKA$_2$ | 13 | 48 |
| M.** spleen cell | 48 | 42 |
| X-ray-thymoma | 20 | 31 |

*Normal human peripheral lymphocytes
**Mouse

Among all tested cells, only Don-6 cells which have no malignancy showed no cap formation. From these results it is supposed that Aloctin A receptors exist in lymphocytes and malignant cells, and by formation of receptor-Aloctin A complex, redistribution of the complex is stimulated.

Reactivity with serum proteins

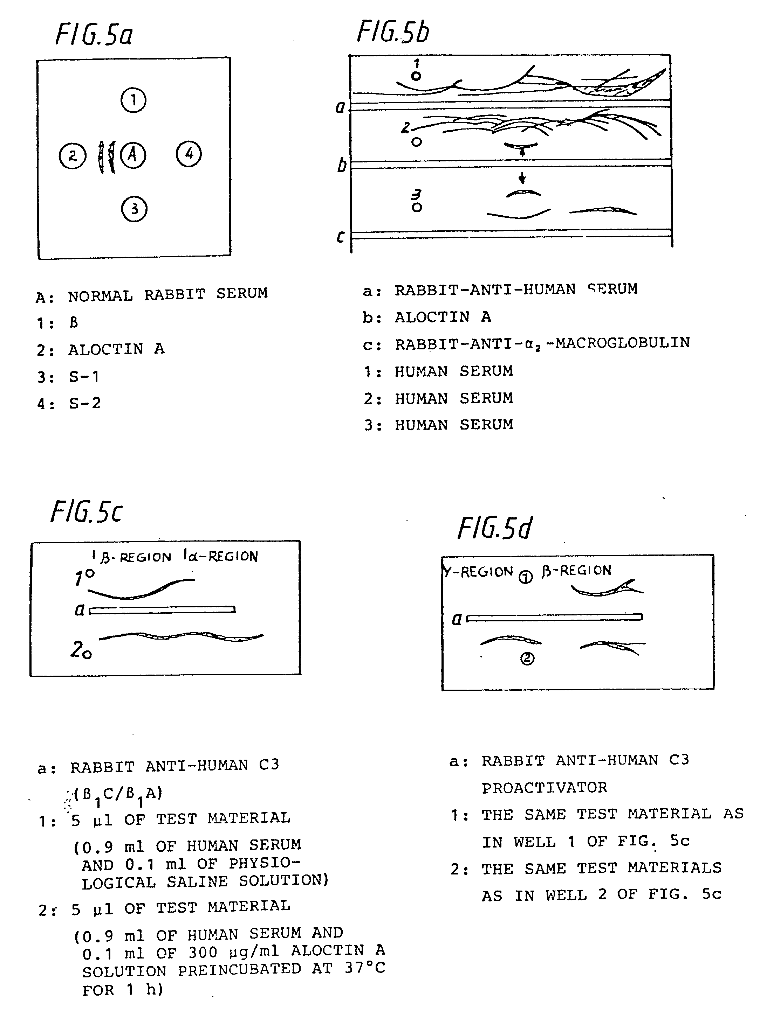

Immunodiffusion was performed by the Ouchterlony method (Ouchterlony, O. (1953) Acta Path. Microbiol Scand. 32, 231—240), using 1.0% Agarose II (Dojin-do Laboratories, Japan) in 0.05 M phosphate buffer, pH 6.5. Immunoelectrophoresis was performed according to the technique of Hirschfeld (Hirschfeld, J. (1960) Sci. Tools 7, 18—25), using 1.4% Agarose II in 0.025 M veronal buffer, pH 8.6. The reactivity of the crude extract of Aloe against various sera by the gel diffusion test (Ouchterlony test) was previously reported (Fujita, K., Suzuki, I., Ochiai, J., Shinpo, K., Inoue, S. and Saito, H. (1978) Experientia 34, 523—524). Sixteen sera of specimens such as human, rabbit, goat, dog, cat, horse, pig, rat, fetal rat, bovine, fetal calf, snake and snapping turtle were tested, and all of them showed positive reactivity. The extract of Aloe reacted not only with mammalian sera but also with fish, amphibia, as well as reptilia. Moreover, more than two precipitin lines were detected, in almost all sera tested. Among purified fractions of P-1 ($\beta$ subunit) and Aloctin A, only Aloctin A reacted with human serum proteins, and two precipitin lines were detected (Fig. 5a). Two precipitin lines mean two different serum proteins reacting with Aloctin A.

Center well: normal rabbit serum, Well 1: $\beta$ (1 mg/ml), Well 2: Aloctin A (1 mg/ml), Well 3: S-1 (1 mg/ml), Well 4: S-2 (1 mg/ml).

Figure 5b shows the immunoelectrophoresis of $\alpha_2$-macroglobulin in human serum. The right is the anode and the left is the cathode. Precipitin reactions were developed against rabbit anti-human serum (upper portion), rabbit anti-human $\alpha_2$-macroglobulin (lower portion), and Aloctin A (middle portion, arrow). The immunoelectrophoretic pattern indicates that the electric mobility of the precipitin line of human serum protein against Aloctin A (arrow) completely coincides with that of $\alpha_2$-macroglobulin. Well 1 and 2: human serum, Trough a: rabbit anti-human serum, Trough b: Aloctin A solution in 0.05 M phosphate buffer (300 $\mu$g/ml), Trough C: rabbit anti-$\alpha_2$-macroglobulin. By the described immunoelectrophoresis using human serum, $\alpha_2$-macroglobulin was found to be one of the serum proteins that reacted with Aloctin A.

Activation of serum complement components by Aloctin A

When the C3 component or the C3 proactivator is activated, there is a change in electrophoretic mobility; C3 component ($\beta$1C) moves to $\beta$ region while activated C3 component ($\beta$1A) shifts to the $\alpha$ region. C3 proactivator is seen in the $\beta$ region in agarose electrophoresis but it moves to the $\gamma$ region when the C3 proactivator is activated. Figures 5c and 5d show the immunoelectrophoretic mobility of the C3 component and the C3 proactivator of human serum incubated with Aloctin A at 37° C for 1 h.

In Fig. 5c the symbols have the following meaning:

Well 1: 5$\mu$l of test material which consists of 0.9 ml of human serum and 0.1 ml of physiological saline solution.

Well 2: 5 $\mu$l of test materials which consist of 0.9 ml of human serum reacted with 0.1 ml of 300 $\mu$g/ml P-2 solution at 37° for 1 h.

Trough (a): rabbit anti-human C3 ($\beta_1$C/$\beta_1$A).

7

In the case of human serum treated with Aloctin A an immunoprecipitin line against rabbit anti-human C3 ($\beta_1$C/$\beta_1$A) was seen in the $\beta$ and $\alpha$ region, whereas, in the non-treated human serum, the immunoprecipitin line was found only in the $\beta$ region.

Fig. 5d shows that precipitin line of human serum treated with Aloctin A against rabbit anti-human C3 proactivator was seen in the $\beta$ and $\gamma$ region, whereas, the precipitin line of non-treated human serum was found only in the $\beta$ region. Immunoelectrophoresis was carried out as described in Fig. 5c. Well 1 contains the same test material as in well 1 of Fig. 5c, and well 2 contains the same test material as in well 2 of Fig. c. Trough: rabbit anti-human C3 proactivator.

It has been well known that a complement system is activated via either the classical or alternate pathway (Sandberg, A. L., Osler, A. G., Shin, H. S., and Oliverira, B. (1970) J. Immunol. *104*, 329—334; Gerwurz, H., Shin, H. S., and Mergenhagen, S. E. (1968) J. Exp. Med. *128*, 1049—1057; Marcus, R. L., Shin, H. S., and Mayer, M. M. (1971) Proc. Natl. Acad. Sci. *68*, 1351—1354; and Frank, M. M., May, J., Gaither, T., and Ellman, L. (1971) J. Exp. Med. *134*, 176—187) and that only $Mg^{++}$ is necessary for the alternate pathway, while both $Mg^{++}$ and $Ca^{++}$ are essential for the classical pathway. We attempted to determine whether Aloctin A acts through the classical or the alternate pathway. To 1 ml of fresh human serum was added 0.1 ml of Aloctin A suspension (1 mg/ml). The mixture was incubated at 37° C for one hour and then centrifuged at 5 000 rpm for 30 min. in the cold. The supernatant was used for immunoelectrophoresis to test the complement activation. The precipitin line of the C3 proactivator converted in the presence of EGTA (ethyleneglycolbis-N,N′-tetra-acetic acid) was seen in the $\gamma$ region, but it was not seen in the presence of EDTA, and activation of complement components depended upon temperature. It was also examined as to whether Aloctin A activates C1, C2 and C4 by the method of Takada et al. (Takada, Y., Arimoto, Y., Mineta, H., and Takada, A., (1978) Immunology *34*, 509—515). The result showed no activation of C1, C2 and C4. These results suggest that the activation of complement components by Aloctin A occurs via the alternate pathway.

### Use as anti-tumor agent

It is clarified that Aloctin A has a strong anti-tumor activity from following experiments.

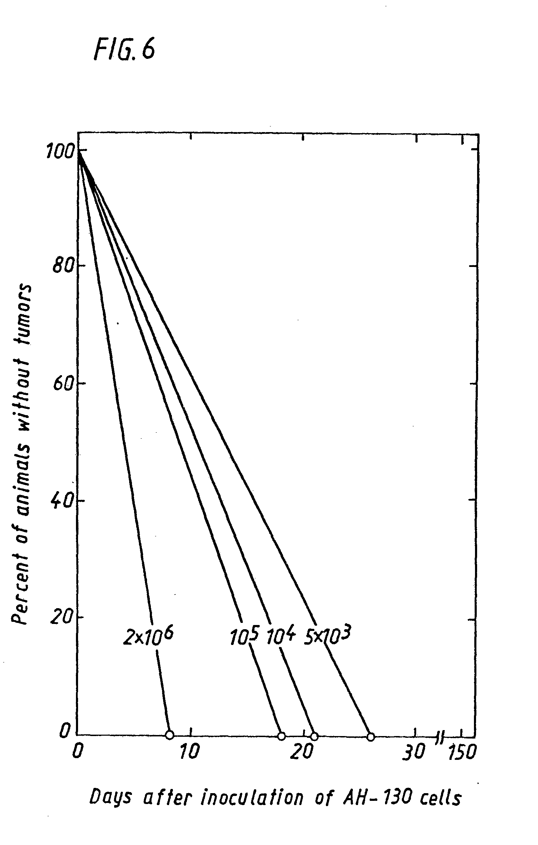

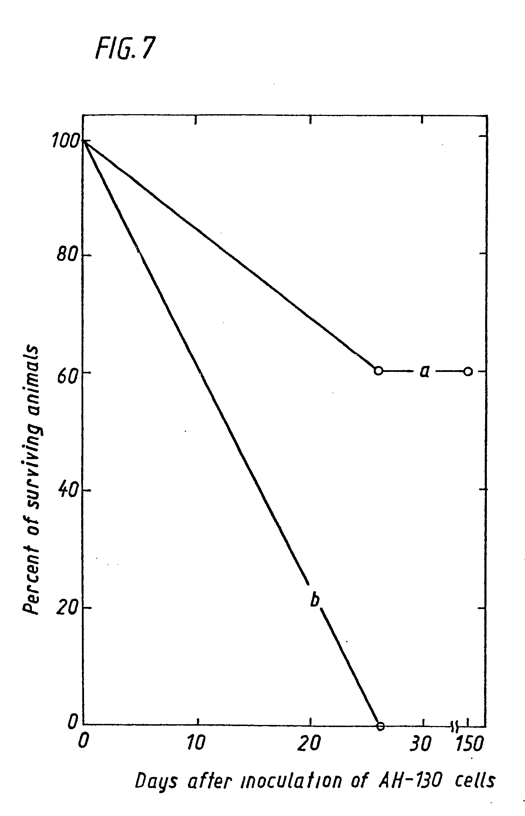

Donryu inbred rats weighing about 100 g were used for the anti-tumor assay. Tumorigenicity of AH-130 cells after intraperitoneal implantation of $5 \times 10^3$ to $2 \times 10^6$ cells into 30 to 40 days old rats was preliminarily examined. All animals were dead with tumor at 8 to 27 days (Fig. 6). Seven days old AH-130 ascites cells, $5 \times 10^3$ cells in 0.5 ml doses, were injected intraperitoneally into rats. Effect on tumor development of Aloe crude preparation, Acidic sup and Acidic ppt, injected at different times before and after tumor cell implantation was examined. The obtained result was shown in Fig. 7 a and b.

a: Rats were intraperitoneally injected with 2 mg of Acidic ppt (2 mg protein) every day for 7 days, and then $5 \times 10^3$ AH-130 cells were implanted at 7 days after last injection of Acidic ppt.

b: Control: Rats were not injected with any Aloe preparation and implanted with $5 \times 10^3$ AH-130 cells.

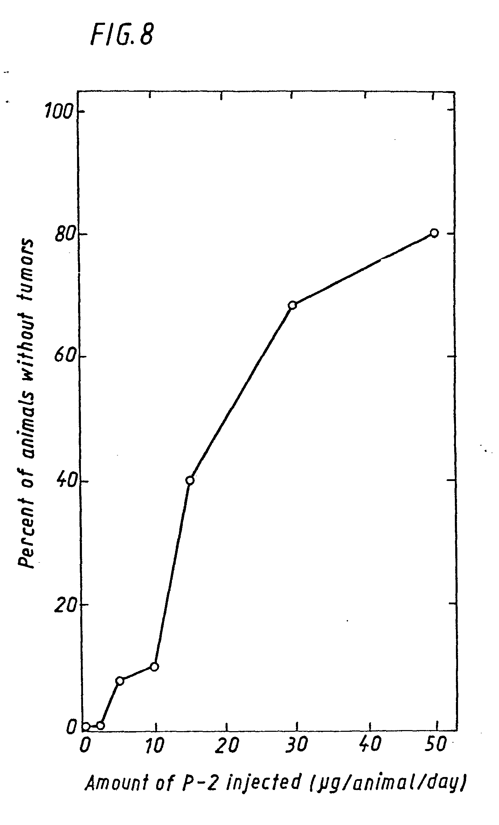

The next experiment was carried out to determine the dose response of Aloctin A to surviving time of tumor cell implanted animals. Various amounts of Aloctin A were injected every day for 7 days from 14 days to 7 days prior to tumor cell implantation, and result from animals was scored at 80 days, that is, at 54 days after the last control animal with tumor had died. The result was shown in Fig. 8. As shown in Fig. 8, the number of surviving animals without tumors, that is the anti-tumor activity of Aloctin A varied depending on the dose. Each percentage in the result was the average of 25—30 animals from three experiments. Furthermore, ten rats, which were injected with 50 $\mu$g of Aloctin A every day for 7 days and survived at 120 days after tumor cell implantation, were re-implanted with $1 \times 10^5$ AH-130 cells, and tumors regressed completely in all tested rats.

On the other hand, JCL-ICR mice weighing about 30 g were used. Mice were injected with $2 \times 10^5$ sarcoma 180 cells maintained as ascites form into the right shoulder subcutane. After the tumor cell implantation, the mice were injected daily with 30 $\mu$g of isotonic solution of Aloctin A for 2 weeks. After 60 days, inhibition ratio was calculated as 80% and complete regression of the tumor was 2/10.

The glycoprotein, Aloctin A is non-toxic, and there is nothing left after the complete regression of the tumors within doses used in the present experiment.

When Aloctin A is used as an active substance in medicines it may be formulated in the usual manner by combining it with a usual carrier (for instance an aqueous carrier) and with usual additives.

### Claim

A glycoprotein isolated from Aloe, a plant belonging to *liliaceae*,

(1) having a molecular weight of $1.8 \times 10^4$;

(2) having a protein to sugar ratio of 8 to 2 by weight;

(3) giving a single band in sodium dodecyl sulfate-polyacrylamide gel electrophoresis without 2-mercaptoethanol treatment, and two discrete bands in sodium dodecyl sulfate-polyacrylamide gel electrophoresis with 2-mercapto ethanol treatment;

(4) having hemagglutinating and cytoagglutinating activity for transformed cells;

(5) having mitogenic activity for lymphocytes;

(6) having binding activity with serum proteins in mammalian, fish, amphibia and reptilia and forming precipitin lines in agarose gel plates; and

(7) having activating activity of complement third component via alternative pathway.

**Patentanspruch**

Aus Aloe, einer Pflanze der Liliaceae, isoliertes Glycoprotein, gekennzeichnet durch

(1) ein Molekulargewicht von $1,8 \times 10^4$;

(2) ein Gewichtsverhältnis von Protein zu Zucker im Bereich von 8 bis 2;

(3) das eine einzige Bande bei der Natrium dodecyl-sulfat-polyacrylamid-Gel-Elektrophorese ohne Behandlung mit 2-Mercaptoethanol und zwei gesonderte Banden bei der Natriumdodecylsulfat-Polyacrylamid-Gel-Electrophorese bei Behandlung mit 2-Mercaptoethanol ergibt;

(4) das hämagglutinierende und cytoagglutinierende Aktivität gegenüber transformierten Zellen aufweist;

(5) das mitogene Aktivität gegenüber Lymphozyten aufweist;

(6) das bindende Aktivität für Serumproteine von Säugetieren, Fischen, Amphibien und Reptilien besitzt und Präzipitin-Linien in Agarosege-Platten bildet; und

(7) aktivierende Aktivität für Komplement-Drittkomponenten über den Alternativ-Mechanismus (Alternativweg) besitzt.

**Revendication**

Glycoproteine, isolé d'Aloe, une plante de Liliaceae;

(1) possédant un poids moléculaire de $1,8 \times 10^4$;

(2) possédant un proportion de poids de proteine à sucre de 8 à 2;

(3) produisant une seule bande dans l'électrophorèse au gel dodecylsulfate de sodium-polyacrylamide sans traitement au 2-mercaptoethanol et deux vandes discrètes dans l'électrophorèse au gel dodecylsulfate de sodium-polyacrylamide avec traitement au 2-mercaptoethanol;

(4) possédant activité hemagglutinante et cyto-agglutinante pour cellules transformées;

(5) possédant activité mitogénétique pour lymphocytes;

(6) possédant activité liante avec des sérum-proteines dans mammifères, poissons, amphibia et reptilia, et produisant de lignes de précipitin dans plaques d'agarose gel, et

(7) possédant activité activant vers le troisième composant du complément à la route alternative.

FIG. 1

# FIG. 2

FIG. 3

a      b      c

FIG.4

# 0 002 452

## FIG.5a

A: NORMAL RABBIT SERUM

1: ß

2: ALOCTIN A

3: S-1

4: S-2

## FIG.5b

a: RABBIT-ANTI-HUMAN SERUM

b: ALOCTIN A

c: RABBIT-ANTI-$\alpha_2$-MACROGLOBULIN

1: HUMAN SERUM

2: HUMAN SERUM

3: HUMAN SERUM

## FIG.5c

a: RABBIT ANTI-HUMAN C3
$(\beta_1 C/\beta_1 A)$

1: 5 µl OF TEST MATERIAL

(0.9 ml OF HUMAN SERUM
AND 0.1 ml OF PHYSIO-
LOGICAL SALINE SOLUTION)

2: 5 µl OF TEST MATERIAL

(0.9 ml OF HUMAN SERUM AND
0.1 ml OF 300 µg/ml ALOCTIN A
SOLUTION PREINCUBATED AT 37°C
FOR 1 h)

## FIG.5d

a: RABBIT ANTI-HUMAN C3
PROACTIVATOR

1: THE SAME TEST MATERIAL AS
IN WELL 1 OF FIG. 5c

2: THE SAME TEST MATERIALS
AS IN WELL 2 OF FIG. 5c

5

FIG. 6

0 002 452

6

FIG. 7

FIG. 8